Europäisches Patentamt

European Patent Office

Office européen des brevets

(19)

(11) Veröffentlichungsnummer: **0 321 815**
**A2**

## (12) EUROPÄISCHE PATENTANMELDUNG

(21) Anmeldenummer: **88120703.9**

(22) Anmeldetag: **10.12.88**

(51) Int. Cl.4: **C07C 127/15 , D06M 15/423**

(30) Priorität: **22.12.87 DE 3743539**

(43) Veröffentlichungstag der Anmeldung:
**28.06.89 Patentblatt 89/26**

(84) Benannte Vertragsstaaten:
**AT BE CH DE ES FR GB IT LI NL SE**

(71) Anmelder: **Chemische Fabrik Pfersee GmbH**
**Färberstrasse 4**
**D-8900 Augsburg(DE)**

(72) Erfinder: **Sandner, Bernhard**
**Ortlerweg 12**
**D-8901 Diedorf(DE)**
Erfinder: **Mangold, Rudolf**
**Eichenstrasse 6**
**D-8901 Adelsried(DE)**

(54) **Verfahren zur Herstellung von veretherten Methylolalkylharnstoffen, die daraus hergestellten Dispersionen und deren Verwendung.**

(57) Die vorliegende Erfindung beschreibt ein Verfahren zur Herstellung von veretherten Methylolalkylharnstoffen durch Umsetzung von Fettaminen mit Harnstoff in etwa molarem Verhältnis unter Ammoniakabspaltung, Methylolierung und Veretherung, wobei im Anschluß an die Veretherung eine Nachveretherung mit Methanol und/oder Ethanol erfolgt.

Die so hergestellten Aktivsubstanzen liefern lager-, schüttel- und scherstabile Dispersionen, die sehr gut als reaktive, hydrophobe Weichmacher für Fasermaterialien einsetzbar sind.

EP 0 321 815 A2

**Verfahren zur Herstellung von veretherten Methylolalkylharnstoffen, die daraus hergestellten Dispersionen und deren Verwendung**

Die vorliegende Erfindung betrifft ein Verfahren zur Herstellung von veretherten Methylolalkylharnstoffen, die durch Nachveretherung weiter modifiziert werden, daraus bereitete besonders stabile Dispersionen und deren Verwendung.

Die Umsetzung von Fettaminen mit Harnstoffen in etwa molarem Verhältnis unter Ammoniakabspaltung zu N-Alkylharnstoffen ist bekannt. Auch die weitere Umsetzung dieser Verbindungen mit Formaldehyd, insbesondere mit Paraformaldehyd gehört zum Stand der Technik ebenso wie die Veretherung der dabei hergestellten Methylolverbindungen mit C3- und C4-Alkoholen (siehe US-PS 2 361 185, DE-PS 762 964, DE-AS 1 934 177 und DE-OS 2 320 302). Die so hergestellten Produkte werden in Dispersionsform überführt und ergeben bei der Behandlung von Fasermaterialien einen angenehmen, weichen, hydrophoben Effekt. Doch läßt die Stabilität der Dispersionen zu wünschen übrig. So zeigen dieselben schon nach relativ kurzer Zeit Stippenbildung, die mit zunehmender Lagerzeit in grießige Abscheidungen und Aufrahmungen übergehen und auch eine Verpastung derselben ist immer wieder zu beobachten. Dabei sind die bekannten Dispersionen auch gegenüber mechanischen Belastungen äußerst empfindlich.

Aufgabe der vorliegenden Erfindung war es nun, diese Nachteile zu beseitigen. Überraschenderweise gelingt das in einfacher Weise dadurch, daß im Anschluß an die Veretherung des Methylol-Alkylharnstoffes mit Methanol und/oder Ethanol nachverethert wird.

Gegenstand der Erfindung ist deshalb ein Verfahren zur Herstellung von veretherten Methylol-Alkylharnstoffen wie es in den Ansprüchen 1 bis 4 beschrieben ist. Daneben werden die aus den Aktivsubstanzen bereiteten Dispersionen und deren Verwendung unter Schutz gestellt.

Die Herstellung der Alkylharnstoffe aus den Alkylaminen und Harnstoffen ist im eingangs genannten Stand der Technik hinreichend beschrieben. Die Ausgangssubstanzen werden dazu in etwa molarem Verhältnis unter Rühren aufgeheizt und unter Ammoniakabspaltung zur Reaktion gebracht. Die Methylolierung kann in einem getrennten Arbeitsgang, bevorzugt aber unmittelbar im Anschluß an die vorstehende Umsetzung dadurch erfolgen, daß in die Schmelze Formaldehyd, insbesondere Paraformaldehyd und gegebenenfalls ein Lösungsmittel eingetragen und im alkalischen Milieu die Methylolierung vorgenommen wird. Im allgemeinen folgt danach die Veretherung mit den C3/C4-Alkoholen in saurem Medium. Selbstverständlich kann der C3/C4-Alkohol schon von vornherein als Lösungsmittel mit zugesetzt werden und die eigentliche Veretherung dann durch Zugabe der Säure eingeleitet werden.

Ebenso wie der Formaldehyd wird auch der C3/C4-Alkohol, vor allem Isopropanol, Butanol und Isobutanol im Überschuß eingesetzt und in Gegenwart von Säure die Veretherung vollzogen. Entscheidend ist dabei, daß die Säuremenge nicht zu niedrig aber auch nicht zu hoch angesetzt wird, da sonst nur eine ungenügende Veretherung abläuft. Die verwendeten Mengen an Säure liegen bei mindestens 0,008 bis 0,03, insbesondere 0,01 -0,025 Mol bezogen auf 1 Mol Fettamin. Wird die zur Veretherung eingesetzte Säuremenge gezielt gewählt, so wird schon durch die dann erfolgende verstärkte Veretherung eine merkliche Stabilitätsverbesserung der aus diesen Produkten hergestellten Dispersionen erzielt.

Aber trotz sorgfältiger Reaktionsführung ist es immer noch nicht möglich - das haben unsere Überprüfungen gezeigt - die Veretherung mit den C3/C4-Alkoholen vollständig ablaufen zu lassen. Es bleiben vermutlich immer noch freie Methylolgruppen übrig, die im Laufe der Zeit vor allem in den daraus bereiteten Disperionen, zu Nebenreaktionen führen. Dadurch aber verändern sich die Dispersionen - Stippen- und Flockenbildung, ungenügende mechanische Stabilität sind die Folge.

Es ist nun ein besonderes Verdienst der vorliegenden Erfindung, diese Nachteile durch eine anschließende Nachveretherung mit Ethanol, insbesondere Methanol beseitigt zu haben, ohne daß die gewünschten hydrophoben Weichgriffeffekte erkennbar beeinflußt werden. Zur Erzielung der gewünschten Nachveretherung werden die genannten Alkohole zugegeben und bei Temperaturen von 75° C bis Rückflußtemperatur in Gegenwart der vorhandenen Säuren (als solche sind insbesondere Mineralsäuren, wie Schwefel-, Salzsäure, aber auch Oxal- und Essigsäure und ähnliche geeignet) nachverethert. Der Alkohol (Ethanol, insbesondere Methanol) wird in Mengen von 1 bis 6, insbesondere 2 bis 5 Mol je Mol Fettamin verwendet und ebenso wie der überschüssige C3/C4-Alkohol nach der Reaktion - insbesondere unter vermindertem Druck - abdestilliert, nachdem vorher der pH-Wert wie bekannt, z.B. durch Zugabe von Alkali oder Alkanolaminen, auf etwa neutral eingestellt worden ist.

Bei dem erfindungsgemäßen Verfahren ist zu beachten, daß durch die verwendete höhere Säuremenge die Veretherung mit den C3/C4-Alkoholen möglichst weitgehend erfolgt und dann mit den niedrigen Alkoholen nachverethert wird. Nur so ist es gewährleistet, daß stabile Disperionen entstehen, die auch die gewünschten Effekte ergeben. Zu beachten ist dabei allerdings weiterhin, daß durch erhöhte Säuremengen,

2

z.B. über 0,03 Mol je Mol Fettamin, die Stabilität der daraus hergestellten Emulsionen, insbesondere die Schüttelstabilität wieder abnimmt, weshalb höhere Säuremengen zu vermeiden sind.

Die Herstellung der Dispersionen gelingt auch in bekannter Weise. Im allgemeinen kann dabei wie folgt vorgegangen werden:

Man legt eine wäßrige Emulgatorlösung vor, erwärmt dieselbe und gibt den veretherten Methylolalkylharnstoff in geschmolzener Form zu (Temperatur 50 bis 70° C).

Anschließend wird eine Hochdruckhomogenisierung durchgeführt. Es ist dabei ohne weiteres möglich, der wäßrigen Lösung Alkohole, Ketone und ähnliches zuzusetzen. Die fertigen Dispersionen enthalten in der Regel 15 bis 35, insbesondere 15 bis 25 Gew.% Aktivsubstanz (veretherten Methylol-alkylharnstoff). Die einsetzbaren Emulgatoren sind bekannt. Als Beispiele seien ethoxilierte Alkohole, Alkylphenole, Amine und Amide (letztere auch in Form ihrer Salze) sowie Polyvinylalkohol genannt.

Diese Dispersionen werden ebenso wie diejenigen nach dem Stand der Technik als reaktive, hydrophobe Weichmacher für Fasermaterialien, insbesondere Textilien in üblichen Mengen (im allgemeinen 0,05 bis 1,5 Gew.% bezogen auf Warengewicht) in ebenfalls bekannter Weise eingesetzt. Selbstverstandlich ist es dabei möglich, andere bekannte Textilhilfsmittel, vor allem Hochveredlungs- und Füllmittel und die dazugehörigen Katalysatoren mitzuverwenden.

Der Gegenstand der vorliegenden Erfindung wird in den nachfolgenden Beispielen naher erläutert. In diesen Beispielen sind unter Teilen = Gewichtsteile und unter Prozentangaben = Gewichtsprozent zu verstehen.

## Beispiel 1

In einem mit Rückflußkühler, Rührer und Thermometer versehenen 2 l-Dreihalskolben werden 487,5 g Stearylamin (mittlere Kettenverteilung: 1 % $C_{12}$, 4 % $C_{14}$, 31 % $C_{16}$, 59 % $C_{18}$, Spuren > $C_{18}$, alles gesättigt, 5 % Oleylamin) vorgelegt und auf 70° C aufgeheizt. Dann werden 108 g Harnstoff zugesetzt und unter Rühren auf 135° C weitergeheizt. Bei dieser Temperatur wird während 3 Stunden gerührt, wobei sich 30,6 g Ammoniakgas abspalten. Anschließend wird der Kolbeninhalt auf 100° C gekühlt, es werden 592 g Isobutanol sowie 232,5 g Paraformaldehyd zugefügt, mit etwa 2 g Triethanolamin wird ein schwach alkalischer pH-Wert eingestellt und dann wieder auf 100° C aufgeheizt. Nach 1 Stunde Rühren bei dieser Temperatur wird die Heizquelle entfernt, dem Ansatz 4 g Schwefelsäure 98 %ig zugefügt und 30 Minuten bei 95° C weitergerührt. Dann werden über Tropftrichter 297 g Methanol zugesetzt und 15 Minuten unter Rückfluß gehalten. Nach kurzem Abkühlen wird mit 12,3 g Natronlauge 50 %ig die Schwefelsäure neutralisiert. Nachdem der Rückflußkühler gegen einen Destillationskühler ausgetauscht wurde, werden im Vakuum bei einer maximalen Innentemperatur von 125° C etwa 950 g Lösungsmittelgemisch, bestehend aus Methanol, Isobutanol, Diisobutylether, Diisobutylformal, Formal und Wasser abdestilliert. Nach dem Abfiltrieren des ausgefällten Glaubersalzes bei etwa 50° C erhält man ca. 750 g einer bei 20° C wachsartigen Masse mit einem Feststoffgehalt von etwa 92 %.

## Beispiel 2

In gleicher Weise wie im Beispiel 1 beschrieben, wird ein Umsetzungsprodukt unter Verwendung von nur 117 g Methanol hergestellt, wobei entsprechend weniger (770 g) Lösungsmittelgemisch abdestilliert wird.

## Beispiel 3

In gleicher Weise wie im Beispiel 1 beschrieben wird ein Umsetzungsprodukt unter Einsatz von nur 400 g Isobutanol und 173 g Methanol bereitet (Destillat 520g).

## Vergleichsbeispiel

Zum Vergleich nach dem Stand der Technik wurde in gleicher Weise wie im Beispiel 1 beschrieben gearbeitet, wobei aber unmittelbar nach der Veretherung mit Isobutanol die Natronlauge zur Neutralisation zugesetzt wird, also die Nachveretherung mit Methanol entfällt. Es wird auch auf diese Weise eine wachsartige Masse mit einem Feststoffgehalt von etwa 92 % erhalten.

Beispiel 4

Herstellung der Dispersionen

In einem 2 l-Becherglas werden 24,8 g Emulgator I (Dodecyloxypropylamin ethoxiliert mit durchschnittlich 12 bis 15 Mol Ethylenoxid, in Form des Acetats), 24,8 g Emulgator II (mit durchschnittlich 15 Mol ethoxiliertes Rizinusöl), 55,6 g Ethylenglykol und 49,4 g Methanol in 300 g Wasser gelöst und auf 65° C erwärmt. Anschließend werden unter dem Schnellrührer 300 g der gemäß den obengenannten Beispielen hergestellten und auf 65° C erwärmten Produkte einturbiniert und auf einer Hochdruckhomogenisiermaschine bei 65 bis 70° C und 300 bar Druck während 10 bis 20 Minuten homogenisiert. Dann wird mit 730 g kaltem Wasser auf Endkonzentration verdünnt. Man erhält etwa 1500 g einer feinteiligen Emulsion mit charakteristischem Geruch und einem Wirksubstanzgehalt von ca. 18,5 %.

Bei der Überprüfung der Stabilität der so hergestellten Dispersionen ergibt sich folgendes Bild:

| Beispiele | 1 (Disp.1) | 2 (Disp.2) | 3 (Disp.3) | Vergleichsbeispiel |
|---|---|---|---|---|
| Schüttelstabilität | | | | |
| Stippen nach 2 h bei 175 Hüben/Min. | keine | keine | keine | gering |
| Scherstabilität | | | | |
| Stippen nach ③Ultraturrax-Behandlung während 1 Min. (Menge 100 ml) | keine | keine | keine | merklich |
| Aussehen nach 4 Wochen Lagerung | keine Stippen und keine Viskositätszunahme | | | deutliche Stippenbildung und geringer Viskositätsanstieg |
| (Ultraturrax eingetragene Marke Janke & Kunkel, D-7813 Staufen) | | | | |

Beispiel 5

Ein mercerisierter, gebleichter und optisch voraufgehellter Baumwollpopelin (ca. 126 g/m²) wird durch Tränken und Abquetschen auf eine Flottenaufnahme von 65 % mit folgender Flotte behandelt:

1 l Wasser enthaltend 40 g der Dispersion (1), 60 g Dimethylolethylenharnstoff und 15 g Magnesiumchlorid hexahydrat. Nach der Foulardierung wird bei 130° C getrocknet und danach während 4 Minuten bei 150° C kondensiert. Es wird ein sehr gut knitterfestes Gewebe mit einem angenehmen weichen oberflächenglatten Griff erhalten.

**Ansprüche**

1. Verfahren zur Herstellung von veretherten Methylolalkylharnstoffen durch Umsetzung von Fettaminen mit Harnstoff in etwa molarem Verhältnis unter Ammoniakabspaltung, Methylolierung und Veretherung in bekannter Weise, dadurch gekennzeichnet, daß die Veretherung mit C3/C4-Alkoholen und die Nachveretherung mit Methanol und/oder Ethanol bei höheren Temperaturen in Gegenwart von Säuren als Veretherungskatalysator durchgeführt wird.

2. Verfahren nach Patentanspruch 1, dadurch gekennzeichnet, daß in Gegenwart von mindestens 0,008 bis 0,03, insbesondere 0,01 bis 0,025 Mol Säure, bezogen auf 1 Mol Fettamin, verethert bzw. nachverethert wird.

3. Verfahren nach Patentanspruch 1 und 2, dadurch gekennzeichnet, daß mit Methanol nachverethert wird.

4

4. Verfahren nach Patentanspruch 1 bis 3, dadurch gekennzeichnet, daß bei Temperaturen von 85°C bis Rückflußtemperatur verethert und bei Temperaturen von 75°C bis Rückflußtemperatur nachverethert wird.

5. Lager-, schüttel- und scherstabile Disperionen der nach den Patentansprüchen 1 bis 4 hergestellten veretherten Methylolalkylharnstoffe.

6. Verwendung der Dispersionen nach Patentanspruch 5 als reaktiver, hydrophober Weichmacher für Fasermaterialien.